# EUROPEAN PATENT APPLICATION

(11) **EP 4 239 074 A1**
(43) Date of publication of application: **06.09.2023**
(21) Application number: 21885299.4
(22) Date of filing: 29.10.2021
(51) Int. Cl.: C12N 15/70, C12N 9/24, C12N 15/56

(54) **RECOMBINANT VECTOR AND EXPRESSION PURIFICATION METHOD FOR HEAT-LABILE UNG FUSION PROTEIN**

(30) Priority: 30.10.2020 CN 202011191136
(71) Applicant: Xiamen University, Xiamen, Fujian 361005 (CN); Xiamen Zeesan Biotech Co., Ltd., Fujian 361101 (CN)
(72) Inventor: ZHANG, Yongyou, Xiamen, Fujian 361102 (CN); WANG, Dan, Xiamen, Fujian 361102 (CN); SONG, Najie, Xiamen, Fujian 361101 (CN)
(74) Representative: Hoppe, Georg Johannes
(86) International application number: PCT/CN2021/127313
(87) International publication number: WO 2022/089573

(57) **Abstract**

Provided are a recombinant vector and an expression purification method for a heat-labile UNG fusion protein. The method comprises: cloning a gene sequence of Cod UNG to a pCold-sumo plasmid to construct a recombinant plasmid pCold-sumo-Cod UNG, transforming the recombinant plasmid into an Escherichia coli BL21 (DE3) competent cell, simultaneously transforming a plasmid pG-Tf2 which expresses chaperone into the competent cell, and carrying out low-temperature-induced expression to obtain a soluble sumo-Cod UNG fusion protein. According to the method, the problem of insoluble Cod UNG expression products is overcome.

## Description

### FIELD OF THE DISCLOSURE

The present disclosure relates to the field of genetic engineering, in particular the present disclosure relates to a method for expressing and purifying a thermolabile UNG fused protein.

### BACKGROUND OF THE DISCLOSURE

Today, much clinical and scientific research relies on detection and quantitative analysis of microanalytes in a limited sample volume, and a real-time quantitative polymerase chain reaction, a digital polymerase chain reaction, and second-generation sequencing are typically used to analyze DNA and RNA. An analysis of small amounts of DNA and RNA molecules in the limited sample volumes (e.g., liquid biopsies and single cells) often requires preamplification to generate a sufficient amount of target molecules, and singleplex quantitations of multiple targets are achieved in a reproducible, specific, and sensitive method. Since the target sequences may require to be amplified in orders of magnitude, handling of subsequent samples causes a potential contamination risk, and the PCR product existing in high concentration may contaminate downstream reactions.

In PCR, deoxythymidine triphosphate (dTTP) can be replaced with deoxyuridine triphosphate (dUTP). Any uracil-containing PCR products can be degraded using uracil DNA N-glycosylase (UNG) before PCR, residual contaminants can be eliminated, and only a thymine-containing target DNA derived from original biological samples is amplified.

Since traditional UNG enzymes cannot be completely deactivated, amplicons can be lost, thereby not facilitating subsequent quantification. Cod UNG prepared in recombinant E. coli strains modified with UNG genes of Atlantic cod can be completely and irreversibly deactivated by moderate heat treatment, and the problem can be avoided.

Three-dimensional structure error of the expressed protein, low expression amount, degradation and impure proteins existing in purified UNG enzyme, and low enzyme activity often occur in the existing methods for expressing and purifying the UNG protein. Therefore, a significant difference exists between an actual production and application and ideal effects of the UNG enzyme.

### BRIEF SUMMARY OF THE DISCLOSURE

The main objective of the present disclosure is to provide a thermolabile UNG fused protein and a method for expressing and purifying thereof. The purified UNG enzyme has high purity, strong activity, no DNase and RNase contamination, and no inhibitory effect on PCR due to easy deactivation.

To achieve the above objective, the present disclosure provides a method for expressing and purifying rCod UNG, it comprises the following steps:

preparing a recombinant vector containing rCod UNG protein, wherein the recombinant vector has a His tag and a SUMO tag;
inducing expression; and
purifying: purifying the fused protein by the His tag to obtain the rCod UNG protein having a high purity.

Preferably, a sequence of the rCod UNG protein is SEQ ID NO. 3; and a sequence of the pCold-SUMO vector is SEQ ID NO. 4.

Preferably, an inducing medium for inducing the expression is 1 weight/volume % NaCl; 1 weight/volume % peptone; and 0.5 weight/volume % yeast extract.

Preferably, conditions while inducing the expression are as follows: OD₆₀₀ is 0.5-0.7, 0.25 mM of IPTG is added, and protein expression is induced for 16 hours at 15 °C and 230 rpm.

Preferably, the purifying comprises lysing strains, extracting the fused protein, and purifying the fused protein.

Preferably, steps of lysing the strains comprise collecting the strains by a first centrifugation of a strain solution obtained by inducing the expression, resuspending by a precooled binding buffer, deconstructing by sonication in an ice-water bath, then performing a second centrifugation, taking a supernatant, performing a third centrifugation, then taking a supernatant, adding polyethyleneimine (PEI) into the supernatant, culturing for 15-20 minutes, and then performing a fourth centrifugation;
Preferably, conditions of the first centrifugation are centrifugating for 10 minutes at 2500 g and 4 °C;
Preferably, conditions of the second centrifugation are centrifugating for 10 minutes at 11000 g and 4 °C;
Preferably, conditions of the third centrifugation are centrifugating for 10 minutes at 1100 g and 4 °C; and
Preferably, conditions of the fourth centrifugation are centrifugating for 15 minutes at 1100 g and 4 °C.

Preferably, a volume ratio of the strain solution obtained by inducing the expression to the binding buffer is 1:20.

Preferably, a power for deconstructing by the sonication is 50%, a frequency is sonicating for 2 seconds and stopping for 3 seconds, and a duration is 20-25 minutes.

Preferably, a volume ratio of the polyethyleneimine to a filtration solution is 1:1000-2000, preferably 1:1000.

Preferably, extraction steps of the fused protein comprise adding a supernatant into an Ni-NTA protein extraction column until all of the supernatant flows through Ni-beeds; then adding a wash buffer having a same volume as the Ni-beeds to wash off impure proteins by repeating1-2 times; and
then adding an elution buffer for an elution, repeating the elution 4-6 times, and collecting all eluates.

Preferably, the purifying the fused protein comprises dialyzing twice with a storage solution for the rCod UNG protein.

According to the design of the applicant, in the present disclosure, a recombinant vector containing rCod UNG gene is prepared to be transformed to the E. coli BL21 (DE3) competent cells containing the molecular chaperone plasmids pG-Tf2. The rCod UNG is properly folded and assembled due to characteristics of the SUMO tag and the molecular chaperone, protein solubilization is stimulated during the purification, and a yield of the rCod UNG protease is improved. The expressed product is purified by the His tag to obtain a product with a higher activity than a natural UNG protease.

According to the present disclosure:
① The rCod UNG expression gene is cloned to the pCold-SUMO vector containing the 6XHis tag and the SUMO tag.
② The pCold-SUMO vector is suitable for inducing the expression at a low temperature, which is beneficial to maintaining an activity of the rCod UNG protease obtained by inducing the expression.
③ The competent cells transformed by the plasmids: the expression vector is directly transformed into the competent cells in a general method, however, in the method of the present disclosure, the competent cells comprise the molecular chaperone plasmids, when the expression is induced, the molecular chaperone is also expressed to help the target protein to be folded and assembled, and protein solubilization is stimulated.
④ In the present disclosure, the SUMO tag and the molecular chaperone do not affect enzyme activity but help the protein to be correctly folded and reduce a wrong three-dimensional structure of the protein, so that an enzyme activity is higher than a wild-type UNG enzyme to a certain extent.

### BRIEF DESCRIPTION OF THE DRAWINGS

FIG. 1 illustrates a view of a pCold-6XHis-SUMO-rCod UNG expression vector of the present disclosure.
FIG. 2 illustrates an SDS-PAGE gel electrophoresis image of rCod UNG mini preparation induced by a general method.
FIG. 3 illustrates an SDS-PAGE gel electrophoresis image of rCod UNG mini preparation induced by the present disclosure.
FIG. 4 illustrates an SDS-PAGE gel electrophoresis image of an rCod UNG protein purified by the present disclosure.
FIG. 5 illustrates an activity assay of the rCod UNG protease purified by the present disclosure.
FIG. 6 illustrates a DNase contamination detection of the rCod UNG protease purified by the present disclosure.
FIG. 7 illustrates an RNase contamination detection of the rCod UNG protease purified by the present disclosure.
FIG. 8 illustrates a deactivation temperature test of the rCod UNG purified by the present disclosure compared with a UNG sold by Thermo Company.
FIG. 9 illustrates a test result of an inhibitory effect on a PCR reaction of an enzyme purified by the present disclosure.
FIG. 10 illustrates a test result of the present disclosure on the digestion ability of rCod UNG protease.

### DETAILED DESCRIPTION OF THE EMBODIMENTS

The embodiments of the present disclosure will be further described below. The examples of the embodiments are illustrated in the drawings, and the same reference characters represent the same elements. The embodiments described in combination with the drawings are used to illustrate the present disclosure, and techniques or conditions unspecified in the embodiments follow the techniques or the conditions described in the articles in the art.

SEQ ID NO: 1 of the following embodiments was synthesized by Sangon Biotech (Shanghai) Co., Ltd., and SEQ ID NO: 2 was synthesized by Suzhou Genewiz Biotechnology Co., Ltd. A nucleotide sequence corresponding to rCod UNG is illustrated in SEQ ID NO: 3, and a pCold-SUMO vector sequence is illustrated in SEQ ID NO: 4.

Restriction endonucleases PstI-HF (Product No. R3140S) and KpnI-HF (Product No. R3142S) were manufactured by NEB, Inc.

PFX DNA polymerase was purified by Yongyou Zhang's research group, School of Life Sciences, Xiamen University.

### Embodiment 1: Construction of pCold-SUMO-rCod UNG vector

A genetic sequence of the rCOD UNG is amplified using PCR. SEQ ID NO: 1 and SEQ ID NO: 2 are used as primers, SEQ ID NO. 3 (synthesized by Sangon (Shanghai) Biotech Co., Ltd.) is used as a template, PFX is used as a DNA polymerase, an amplification system is 50 µL, amplification conditions are 98 °C for 2 minutes; 98 °C for 15 seconds; 64 °C for 15 seconds; 72 °C for 15 seconds; and 72 °C for 2 minutes, a total is 35 cycles, and a PCR product 1 is obtained.

An electrophoresis is performed using a 1.2% (w/v) agarose gel, a target strip is cut, and a gel is recovered to obtain a product 1.

3 µg of pCold-SUMO plasmid is digested with 20 U of each of dual-enzyme-fragmentation vector pCold-SUMO (SEQ ID NO: 4), PstI-HF and KpnI-HF and reacted in a water bath at 37 °C for 4 hours, an electrophoresis is performed using a 1.2% agarose gel, a target strip is then cut, a gel is recovered to obtain a product 2, the enzyme-fragmentation product is transformed into TOP10 competent cells (purchased from Shanghai Weidi Biotechnology Co., Ltd., product No. DL1010) and cultured overnight after coating to a plate to observe whether the enzyme digestion is complete.

A medium for inducing expression is 1 w/v% of NaCl; 1 w/v% of tryptone; and 0.5 w/v% yeast extract. Conditions for inducing the expression are that OD₆₀₀ is 0.5-0.7, and 0.25 mM of IPTG is added and cultured for 16 hours at 15 °C and 230 rpm.

A recombinant plasmid clone is performed using a ligase-independent ligation reaction, and a reaction system is as follows: 30 ng of a recovery product (the product 2) of the dual-enzyme-fragmentation vector pCold-SUMO, 20 ng of a recovery product (the product 1) of rCod-UNG PCR, 20 U of EoxIII (purchased from TAKARA Inc.), 1 µL of 10X EoxIII reaction buffer, and water is added to yield 10 µL. After a reaction is performed in ice bath for 1 hour, 1 µL of 0.5 M EDTA (pH 8.0) is added, reacted at 65 °C for 10 minutes, and inserted back into ice for later use.

100 µL of the TOP10 competent cells is taken, melted on ice, and added to the above-mentioned ligation product, heat shock is performed at 42 °C for 90 seconds after ice bath for 30 minutes, it is inserted back into ice, left to stand for 2 minutes, coated on an ampicillin LB medium, and placed upside-down overnight in a constant temperature incubator at 37 °C.

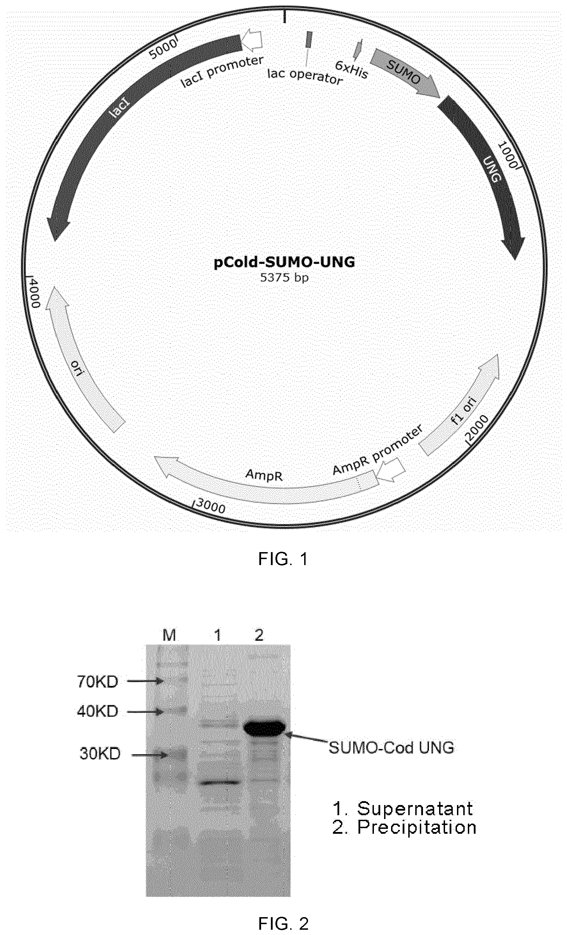

A single clone is picked, the bacteria is shaken for 12 hours at 37 °C and 200 rpm using 5 mL of an ampicillin medium, 1 µL of a bacterial solution is taken for PCR, a portion in which the electrophoresis is correct is sent for sequencing (Xiamen Borui Biotechnology Co., Ltd.), a name of a portion in which sequencing is correct is referred to as pCold-6XHis-SUMO-rCod-UNG, and a view of the vector is illustrated in FIG. 1.

The expression vector pCold-6XHis-SUMO-rCod-UNG is extracted from the rest of the bacterial solution using a plasmid mini kit (purchased from Tiangen Biotech Co., Ltd., product No. DP103-03).

### Embodiment 2: Small expression of the 6XHis-SUMO-rCod-UNG

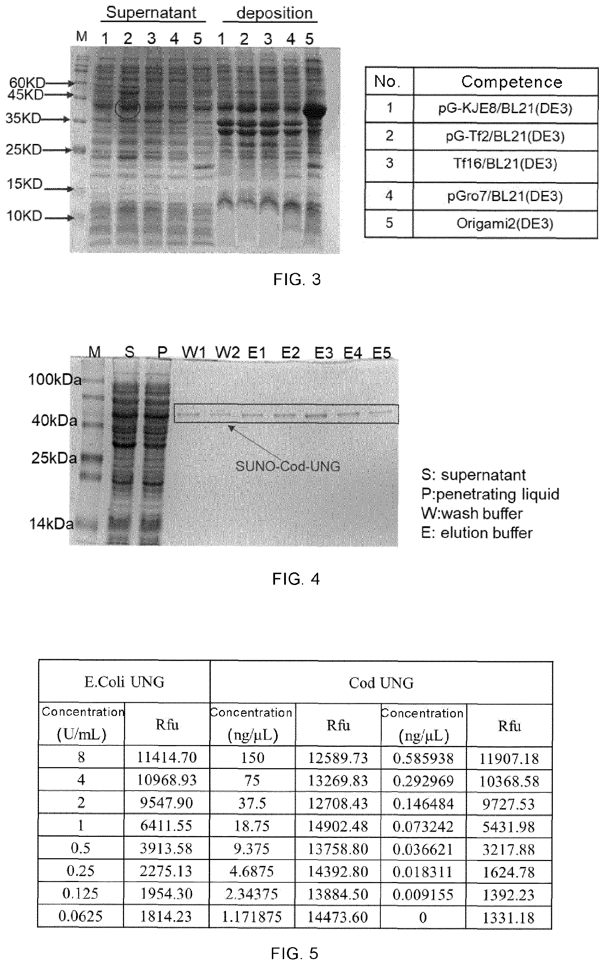

A preparation of a strain of the pCold-6XHis-SUMO-rCod-UNG expression vector: 25 ng of the pCold-6XHis-SUMO-rCod-UNG expression vector obtained in Embodiment 1 is added to 100 µL of 5 kinds of BL21(DE3) competent cells (purchased from Novo(Shanghai) Scientific, Inc.) containing different molecular chaperone plasmids (see FIG. 3), heat shock is performed at 42 °C for 90 seconds after an ice bath for 30 minutes, it is inserted back into ice and left to stand for 2 minutes, coated on a dual-resistant LB medium for ampicillin (30 µg/mL) and chloramphenicol (10 µg/mL), and placed upside-down overnight in a constant temperature incubator at 37 °C.

Comparative embodiment: a pCold-6XHis-SUMO-rCod-UNG expression strain is prepared by a general method

The preparation of the strain of the pCold-6XHis-SUMO-rCod-UNG expression vector is as follows: 25 ng of the pCold-6XHis-SUMO-rCod-UNG expression vector obtained in Embodiment 1 is added to 100 µL of BL21(DE3) competence (purchased from Invitrogen), heat shock is performed at 42 °C for 90 seconds after an ice bath for 30 minutes, it is inserted back into ice and left to stand for 2 minutes, coated on a dual-resistant LB medium for ampicillin (30 µg/ml) and chloramphenicol (10 µg/ml), and placed upside-down overnight in a constant temperature incubator at 37 °C.

A single clone is picked, 5 mL of a dual-resistant LB medium for the ampicillin (30 µg/mL) and the chloramphenicol (10 µg/mL) is used, cultured for 12 hours at 37 °C and 200 rpm, transferred to 5 mL of a dual-resistant LB medium for the ampicillin (30 µg/mL) and the chloramphenicol (10 µg/mL), cultured until OD₆₀₀ is between 0.5-0.7, a stain solution is taken out and cooled on ice for 5 minutes in advance, IPTG is then added to yield 0.25 mM as a working concentration, and it is induced overnight at 15 °C.

### An extraction of rCod-UNG in a small amount:

10 Strain lysis: 5 mL of the overnight strain solution is taken and centrifuged for 15 minutes at 2500 g and 4 °C to collect the strains after balance. It is frozen at -80 °C for 30 minutes, frozen strains are taken out, 0.5 mL of precooled His tag Binding buffer (50 mM of Tris-HCl (pH8.0), 0.5 M of NaCl, 5 mM of imidazole, 10% of glycerol (v/v), 1 mM of PMSF, and 0.2 mM β-ME) is used to fully resuspend the strains (a volume of a strain solution obtained by inducing the expression to a volume of the His tag Binding buffer is equal to 10:1), it is deconstructed by sonication in an ice-water bath, a power is 10%, a frequency is sonicating for 2 seconds and stopping for 3 seconds, and a duration is 5 minutes.

It is centrifuged for 10 minutes at 11000 g and 4 °C, 40 µL of a supernatant is taken, and 10 µL of a 5XSDS-PAGE loading buffer (Tris-HCl (pH 6.8) (50 mM); SDS (2%); bromophenol blue (0.1%); glycerol (25%); and β-mercaptoethanol (14.4 mM) is added, a precipitate is resuspended with a 1X SDS-PAGE loading buffer, the protein is denatured at 95 °C for 10 minutes and then inserted back into ice for later use.

10 µL of each sample of the protein supernatant and the precipitate obtained in the small amount in Embodiment 2 and the comparative Embodiment is taken, and an SDS-PAGE electrophoresis is performed, an upper gel is 90 V for 20 minutes; and a lower gel is 150 V for 50 minutes.

Coomassie brilliant blue staining, the gel after electrophoresis is taken, 30 mL of the Coomassie brilliant blue (100 mg of Coomassie brilliant blue R 250 is dissolved in 500 mL of methanol, 100 mL of glacial acetic acid is added, and water is refilled to 1 L) is added and stained for 1 hour at room temperature, and then 40 mL of a destaining solution (methanol to acetic acid to water is equal to 5:1:4) is added, and destained at room temperature, the destaining solution is replaced every 30 minutes and destained 3-4 times, and the results are illustrated in FIG. 3. Referring to FIG. 3, a protein solubility of the rCod UNG protein expression strain prepared by the present disclosure is obviously improved after inducing the expression.

### Embodiment 3: Purification of the 6XHis-SUMO-rCod-UNG

① Inducing the expression in a large amount of a protein: the pCold-6XHis-SUMO-rCod-UNG expression strain obtained in Embodiment 2 is cultured overnight to obtain a seed solution and transferred into 1 L of a liquid LB containing ampicillin (30 µg/mL) and the chloramphenicol (10 µg/mL) at a ratio of 1:100, cultured on a shaker at 37 °C and 200 rpm until OD₆₀₀ is 0.5-0.7, and taken out and cooled on ice for 15 minutes, IPTG is then added to a final concentration of 0.25 mM, and it is placed on the shaker at 15 °C and 200 rpm and cultured for 16 hours for inducing the expression of the protein.

② Strain Lysis: the strains are collected by a first centrifugation, and the collected cells are frozen at -80 °C for more than 30 minutes, and the frozen strains are taken out to resuspend the cells with 50 mL of precooled His-tag Binding buffer (a volume of a strain solution obtained by inducing the expression to a volume of the His tag Binding buffer is equal to 20:1). The cells are deconstructed by sonication in an ice-water bath with 50% power and sonicating for a total of 20 minutes by sonicating for 2 seconds and stopping for 3 seconds. After the sonication is complete, a second centrifugation is performed, a supernatant is taken after the centrifugation, a third centrifugation is performed, a supernatant is transferred to another clean centrifuge tube, polyethyleneimine (PEI) is added (from Energy Chemical, No.: D110141, lot No.: E8NRD7RK, the polyethyleneimine to the supernatant is 1:1000) while stirring, it is put on ice for 15-20 minutes, and a fourth centrifugation is performed after uniform mixing;

Conditions of the first centrifugation are centrifugation for 10 minutes at 2500 g and 4 °C;

Conditions of the second centrifugation are centrifugation for 10 minutes at 11000 g and 4 °C;

Conditions of the third centrifugation are centrifugation for 10 minutes at 1100 g and 4 °C;

Conditions of the fourth centrifugation are centrifugation for 15 minutes at 1100 g and 4 °C;

③ Purification: 1 mL of Ni-NTA agarose (Lot No.160042951) is added into a filter column, 10 mL of each of an Elution buffer (50 mM of Tris-HCl (pH 8.0), 0.5 M of NaCl, 250 mM of imidazole, 10% glycerol (v/v), 1 mM of PMSF, and 0.2 mM of β-ME), a wash buffer (50 mM of Tris-HCl (pH 8.0), 0.5 M of NaCl, 25 mM of imidazole, and 10% glycerol (v/v)) and a His tag Binding buffer are respectively used to wash the Ni-NTA column, the supernatant is added to flow through the Ni-NTA agarose, 2 mL of the wash buffer is then added to elute an impure protein, and 2 mL of an elution buffer is finally used to elute a target protein four times.

After the obtained protein is tested by SDS-PAGE, transferred to a dialysis bag to be dialyzed at 4 °C overnight, dialyzed for another 4 hours after a replacement of new dialysate, and taken out from the dialysis bag, glycerol is added until a final concentration is 50%. A protein concentration is tested by a BCA method (a BCA protein quantification kit is purchased from Sangon Biotech (Shanghai) Co., Ltd., product Nb. E713DA0001), it is diluted as needed for long storage at -80 °C. Electrophoresis results of the SDS-PAGE are illustrated in FIG. 4, and no obvious degradation and generation of impure proteins are observed in the rCod UNG purified by the present disclosure.

An activity test of the rCod UNG purified by the present disclosure is performed, and the results are illustrated in FIG. 5.

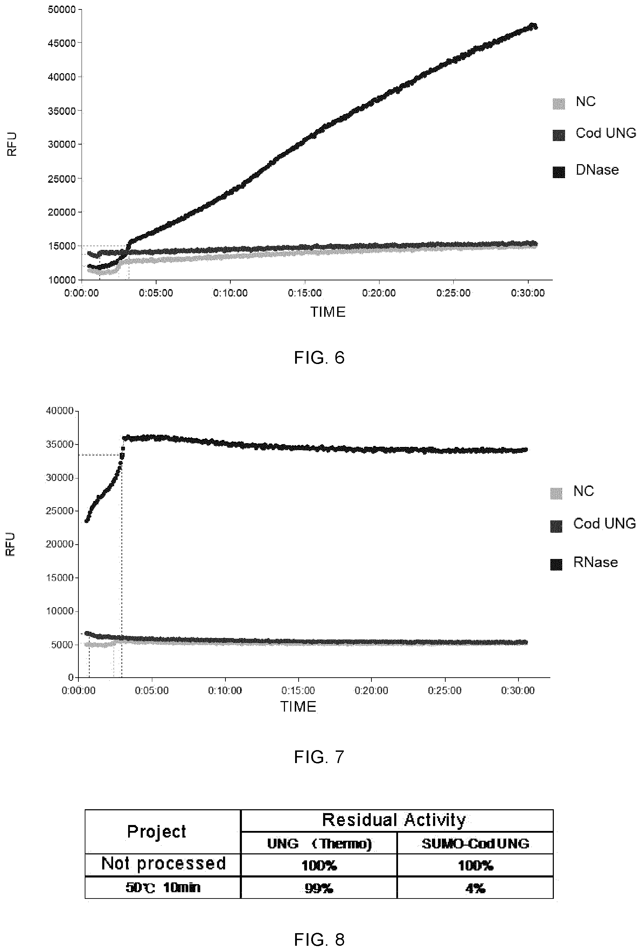

A DNase contamination test of the rCod UNG purified by the present disclosure is performed, and the results are illustrated in FIG. 6.

An RNase contamination test of the rCod UNG purified by the present disclosure is performed, and the results are illustrated in FIG. 7.

A deactivation temperature test of the rCod UNG purified by the present disclosure is performed. An activity test is performed after the UNG is placed at 50 °C for 10 minutes or left untreated, it is compared with a deactivation temperature of a commercially available UNG enzyme (Thermo), and the results are illustrated in FIG. 8.

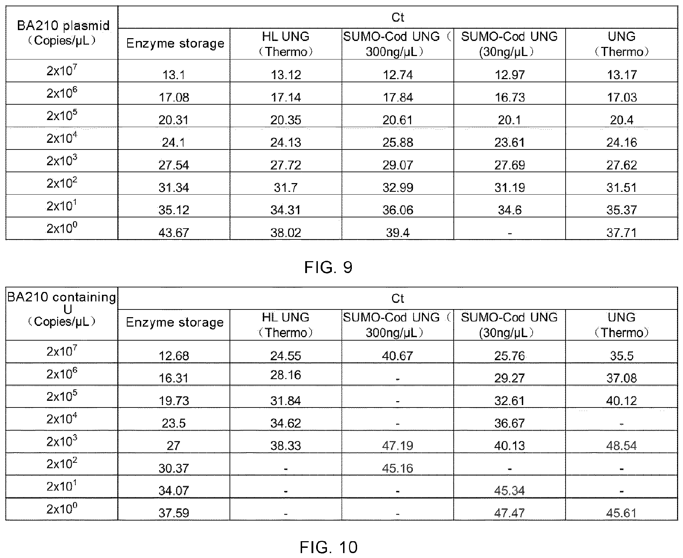

A PCR inhibition ability of the rCod UNG purified by the present disclosure is tested, and the results are illustrated in FIG. 9.

A digestion ability of the rCod UNG purified by the present disclosure is tested, and a template is a purified PCR product containing U, and the results are illustrated in FIG. 10.

Therefore, the present disclosure establishes a method for expressing thermolabile UNG protein and solves an insolubility problem of the expression of the thermolabile UNG protein. The purified SUMO-UNG is tested using nuclease contamination, the thermal deactivation temperature, the PCR inhibition ability, and the digestion ability. It can be seen that the rCod UNG enzyme expressed and purified by the present disclosure is soluble, free from DNase and RNase contamination, active, thermolabile, easily removed from the reaction, and has no inhibitory effect on the PCR reaction.

Sequence listing

SEQ ID NO:1
tagtgagctcggtaccATGttcggagagacttggag
SEQ ID NO:2
Acctatctagactgcattagagtgctctccagttta
SEQ ID NO:3
ATGttcggagagacttggagaagagagctggctgcagagtttgaaaagccatacttcaaacaattgatgtc
ctttgtagctgatgagaggagccgtcacaccgtctacccaccggctgatcaagtgtacagttggacagagatgtgtga
cattcaagatgtgaaagtagtgattctaggccaggacccttaccacggtcccaaccaagcacatggactctgtttcagt
gtgcaaaagccagttccccctccccccagtctcgtgaacatatacaaagaattgtgtaccgacattgatggcttcaagc
atcctggacatggagatctaagcggatgggcaaaacaaggggtgctgctgcttaacgcggtgctgaccgtgcgggc
ccatcaggccaactcccacaaggacagaggctgggagaccttcaccgacgctgtgatcaagtggctgagcgtcaa
ccgggaaggagtggttttcctgttgtggggctcatacgcccataagaagggagcgaccatcgacaggaaacgtcac

catgtcttgcaagctgttcatccatctcctttgtctgctcatcgtgggttccttggttgtaagcacttctccaaggctaacg
ggctgctgaaactatctgggacggagcctataaactggagagcactctaa

SEQ ID NO:4
aaggaatggtgtggccgattaatcataaatatgaaaaataattgttgcatcacccgccaatgcgtggcttaat
gcacatcaaattgtgagcggataacaatttgatgtgctagcgcatatccagtgtagtaaggcaagtcccttc
aagagttatcgttgatacccctcgtagtgcacattcctttaacgcttcaaaatctgtaaagcacgccatatcgc
cgaaaggcacacttaattattaagaggtaatacaccatgaatcacaaagtgcatcatcatcatcatcatatcg
aaggtaggcatatgggcggtggcggtagcggcggtggcggtagcggtatgtcggactcagaagtcaatc
aagaagctaagccagaggtcaagccagaagtcaagcctgagactcacatcaatttaaaggtgtccgatgg
atcttcagagatcttcttcaagatcaaaaagaccactcctttaagaaggctgatggaagcgttcgctaaaag
acagggtaaggaaatggactccttaagattcttgtacgacggtattagaattcaagctgatcagacccctga
agatttggacatggaggataacgatattattgaggctcacagagaacagattggtggtactagtgagctcg
gtaccctcgagggatccgaattcaagcttgtcgacctgcagtctagataggtaatctctgcttaaaagcaca
gaatctaagatccctgccatttggcggggatttttttatttgttttcaggaaataaataatcgatcgcgtaataa
aatctattattatttttgtgaagaataaatttgggtgcaatgagaatgcgcaggccctttcgtctcgcgcgtttc
ggtgatgacggtgaaaacctctgacacatgcagctcccggagacggtcacagcttgtctgtaagcggatg
ccgggagcagacaagcccgtcagggcgcgtcagcgggtgttggcgggtgtcggggctggcttaactat
gcggcatcagagcagattgtactgagagtgcaccataaaattgtaaacgttaatattttgttaaaattcgcgtt
aaatttttgttaaatcagctcattttttaaccaataggccgaaatcggcaaaatcccttataaatcaaaagaata
gcccgagatagggttgagtgttgttccagtttggaacaagagtccactattaaagaacgtggactccaacgt
caaagggcgaaaaaccgtctatcagggcgatggcccactacgtgaaccatcacccaaatcaagttttttgg
ggtcgaggtgccgtaaagcactaaatcggaaccctaaagggagcccccgatttagagcttgacggggaa
agccggcgaacgtggcgagaaaggaagggaagaaagcgaaaggagcgggcgctagggcgctggca
agtgtagcggtcacgctgcgcgtaaccaccacacccgccgcgcttaatgcgccgctacagggcgcgtac
tatggttgctttgacgtatgcggtgtgaaataccgcacagatgcgtaaggagaaaataccgcatcaggcgt
caggtggcacttttcggggaaatgtgcgcggaacccctatttgtttatttttctaaatacattcaaatatgtatc
cgctcatgagacaataaccctgataaatgcttcaataatattgaaaaaggaagagtatgagtattcaacattt
ccgtgtcgcccttattcccttttttgcggcattttgccttcctgtttttgctcacccagaaacgctggtgaaagta
aaagatgctgaagatcagttgggtgcacgagtgggttacatcgaactggatctcaacagcggtaagatcct
tgagagttttcgccccgaagaacgttttccaatgatgagcacttttaaagttctgctatgtggcgcggtattat
cccgtattgacgccgggcaagagcaactcggtcgccgcatacactattctcagaatgacttggttgagtac
tcaccagtcacagaaaagcatcttacggatggcatgacagtaagagaattatgcagtgctgccataaccat
gagtgataacactgcggccaacttacttctgacaacgatcggaggaccgaaggagctaaccgcttttttgc

acaacatgggggatcatgtaactcgccttgatcgttgggaaccggagctgaatgaagccataccaaacga
cgagcgtgacaccacgatgcctgtagcaatggcaacaacgttgcgcaaactattaactggcgaactactta
ctctagcttcccggcaacaattaatagactggatggaggcggataaagttgcaggaccacttctgcgctcg
gcccttccggctggctggtttattgctgataaatctggagccggtgagcgtgggtctcgcggtatcattgca
gcactggggccagatggtaagccctcccgtatcgtagttatctacacgacggggagtcaggcaactatgg
atgaacgaaatagacagatcgctgagataggtgcctcactgattaagcattggtaactgtcagaccaagttt
actcatatatactttagattgatttaaaacttcatttttaatttaaaaggatctaggtgaagatcctttttgataatct
catgaccaaaatcccttaacgtgagttttcgttccactgagcgtcagaccccgtagaaaagatcaaaggatc
ttcttgagatcctttttttctgcgcgtaatctgctgcttgcaaacaaaaaaaccaccgctaccagcggtggttt
gtttgccggatcaagagctaccaactctttttccgaaggtaactggcttcagcagagcgcagataccaaata
ctgttcttctagtgtagccgtagttaggccaccacttcaagaactctgtagcaccgcctacatacctcgctct
gctaatcctgttaccagtggctgctgccagtggcgataagtcgtgtcttaccgggttggactcaagacgata
gttaccggataaggcgcagcggtcgggctgaacggggggttcgtgcacacagcccagcttggagcgaa
cgacctacaccgaactgagatacctacagcgtgagctatgagaaagcgccacgcttcccgaagggagaa
aggcggacaggtatccggtaagcggcagggtcggaacaggagagcgcacgagggagcttccagggg
gaaacgcctggtatctttatagtcctgtcgggtttcgccacctctgacttgagcgtcgatttttgtgatgctcgt
caggggggcggagcctatggaaaaacgccagcaacgcggcctttttacggttcctggccttttgctggcc
ttttgctcacatagtcatgccccgcgcccaccggaaggagctgactgggttgaaggctctcaagggcatc
ggtcgagatcccggtgcctaatgagtgagctaacttacattaattgcgttgcgctcactgcccgctttccagt
cgggaaacctgtcgtgccagctgcattaatgaatcggccaacgcgcggggagaggcggtttgcgtattg
ggcgccagggtggtttttcttttcaccagtgagacgggcaacagctgattgcccttcaccgcctggccctg
agagagttgcagcaagcggtccacgctggtttgccccagcaggcgaaaatcctgtttgatggtggttaac
ggcgggatataacatgagctgtcttcggtatcgtcgtatcccactaccgagatatccgcaccaacgcgcag
cccggactcggtaatggcgcgcattgcgcccagcgccatctgatcgttggcaaccagcatcgcagtggg
aacgatgccctcattcagcatttgcatggtttgttgaaaaccggacatggcactccagtcgccttcccgttcc
gctatcggctgaatttgattgcgagtgagatatttatgccagccagccagacgcagacgcgccgagacag
aacttaatgggcccgctaacagcgcgatttgctggtgacccaatgcgaccagatgctccacgcccagtcg
cgtaccgtcttcatgggagaaaataatactgttgatgggtgtctggtcagagacatcaagaaataacgccg
gaacattagtgcaggcagcttccacagcaatggcatcctggtcatccagcggatagttaatgatcagccca
ctgacgcgttgcgcgagaagattgtgcaccgccgctttacaggcttcgacgccgcttcgttctaccatcga
caccaccacgctggcacccagttgatcggcgcgagatttaatcgccgcgacaatttgcgacggcgcgtg
cagggccagactggaggtggcaacgccaatcagcaacgactgtttgcccgccagttgttgtgccacgcg
gttgggaatgtaattcagctccgccatcgccgcttccactttttcccgcgttttcgcagaaacgtggctggcc
tggttcaccacgcgggaaacggtctgataagagacaccggcatactctgcgacatcgtataacgttactgg

tttcacattcaccaccctgaattgactctcttccgggcgctatcatgccataccgcgaaaggttttgcgccatt
cgatggtgtccgggatctcgacgctctcccttatgcgactcctgcattaggaagcagcccagtagtaggtt
gaggccgttgagcaccgccgccgc

SEQ ID NO:5
aaggaatggtgtggccgattaatcataaatatgaaaaataattgttgcatcacccgccaatgcgtggcttaatgc
acatcaaattgtgagcggataacaatttgatgtgctagcgcatatccagtgtagtaaggcaagtcccttcaagagttatc
gttgatacccctcgtagtgcacattcctttaacgcttcaaaatctgtaaagcacgccatatcgccgaaaggcacacttaa
ttattaagaggtaatacaccatgaatcacaaagtgcatcatcatcatcatcatatcgaaggtaggcatatgggcggtgg
cggtagcggcggtggcggtagcggtatgtcggactcagaagtcaatcaagaagctaagccagaggtcaagccaga
agtcaagcctgagactcacatcaatttaaaggtgtccgatggatcttcagagatcttcttcaagatcaaaaagaccact
cctttaagaaggctgatggaagcgttcgctaaaagacagggtaaggaaatggactccttaagattcttgtacgacggt
attagaattcaagctgatcagacccctgaagatttggacatggaggataacgatattattgaggctcacagagaacag
attggtggtactagtgagctcggtaccATGttcggagagacttggagaagagagctggctgcagagtttgaaaag
ccatacttcaaacaattgatgtcctttgtagctgatgagaggagccgtcacaccgtctacccaccggctgatcaagtgt
acagttggacagagatgtgtgacattcaagatgtgaaagtagtgattctaggccaggacccttaccacggtcccaacc
aagcacatggactctgtttcagtgtgcaaaagccagttccccctccccccagtctcgtgaacatatacaaagaattgtg
taccgacattgatggcttcaagcatcctggacatggagatctaagcggatgggcaaaacaaggggtgctgctgctta
acgcggtgctgaccgtgcgggcccatcaggccaactcccacaaggacagaggctgggagaccttcaccgacgct
gtgatcaagtggctgagcgtcaaccgggaaggagtggttttcctgttgtggggctcatacgcccataagaagggagc
gaccatcgacaggaaacgtcaccatgtcttgcaagctgttcatccatctcctttgtctgctcatcgtgggttccttggttg
taagcacttctccaaggctaacgggctgctgaaactatctgggacggagcctataaactggagagcactctaatgca
gtctagataggtaatctctgcttaaaagcacagaatctaagatccctgccatttggcggggatttttttatttgttttcagga
aataaataatcgatcgcgtaataaaatctattattatttttgtgaagaataaatttgggtgcaatgagaatgcgcaggccc
tttcgtctcgcgcgtttcggtgatgacggtgaaaacctctgacacatgcagctcccggagacggtcacagcttgtctgt
aagcggatgccgggagcagacaagcccgtcagggcgcgtcagcgggtgttggcgggtgtcggggctggcttaac
tatgcggcatcagagcagattgtactgagagtgcaccataaaattgtaaacgttaatattttgttaaaattcgcgttaaatt
tttgttaaatcagctcattttttaaccaataggccgaaatcggcaaaatcccttataaatcaaaagaatagcccgagata
gggttgagtgttgttccagtttggaacaagagtccactattaaagaacgtggactccaacgtcaaagggcgaaaaacc
gtctatcagggcgatggcccactacgtgaaccatcacccaaatcaagttttttggggtcgaggtgccgtaaagcacta
aatcggaaccctaaagggagcccccgatttagagcttgacggggaaagccggcgaacgtggcgagaaaggaagg
gaagaaagcgaaaggagcgggcgctagggcgctggcaagtgtagcggtcacgctgcgcgtaaccaccacaccc
gccgcgcttaatgcgccgctacagggcgcgtactatggttgctttgacgtatgcggtgtgaaataccgcacagatgc
gtaaggagaaaataccgcatcaggcgtcaggtggcacttttcggggaaatgtgcgcggaacccctatttgtttatttttc

taaatacattcaaatatgtatccgctcatgagacaataaccctgataaatgcttcaataatattgaaaaaggaagagtat
gagtattcaacatttccgtgtcgcccttattcccttttttgcggcattttgccttcctgtttttgctcacccagaaacgctggt
gaaagtaaaagatgctgaagatcagttgggtgcacgagtgggttacatcgaactggatctcaacagcggtaagatcc
ttgagagttttcgccccgaagaacgttttccaatgatgagcacttttaaagttctgctatgtggcgcggtattatcccgtat
tgacgccgggcaagagcaactcggtcgccgcatacactattctcagaatgacttggttgagtactcaccagtcacag
aaaagcatcttacggatggcatgacagtaagagaattatgcagtgctgccataaccatgagtgataacactgcggcc
aacttacttctgacaacgatcggaggaccgaaggagctaaccgcttttttgcacaacatgggggatcatgtaactcgc
cttgatcgttgggaaccggagctgaatgaagccataccaaacgacgagcgtgacaccacgatgcctgtagcaatgg
caacaacgttgcgcaaactattaactggcgaactacttactctagcttcccggcaacaattaatagactggatggagg
cggataaagttgcaggaccacttctgcgctcggcccttccggctggctggtttattgctgataaatctggagccggtga
gcgtgggtctcgcggtatcattgcagcactggggccagatggtaagccctcccgtatcgtagttatctacacgacgg
ggagtcaggcaactatggatgaacgaaatagacagatcgctgagataggtgcctcactgattaagcattggtaactgt
cagaccaagtttactcatatatactttagattgatttaaaacttcatttttaatttaaaaggatctaggtgaagatcctttttga
taatctcatgaccaaaatcccttaacgtgagttttcgttccactgagcgtcagaccccgtagaaaagatcaaaggatctt
cttgagatcctttttttctgcgcgtaatctgctgcttgcaaacaaaaaaaccaccgctaccagcggtggtttgtttgccgg
atcaagagctaccaactctttttccgaaggtaactggcttcagcagagcgcagataccaaatactgttcttctagtgtag
ccgtagttaggccaccacttcaagaactctgtagcaccgcctacatacctcgctctgctaatcctgttaccagtggctg
ctgccagtggcgataagtcgtgtcttaccgggttggactcaagacgatagttaccggataaggcgcagcggtcggg
ctgaacggggggttcgtgcacacagcccagcttggagcgaacgacctacaccgaactgagatacctacagcgtga
gctatgagaaagcgccacgcttcccgaagggagaaaggcggacaggtatccggtaagcggcagggtcggaaca
ggagagcgcacgagggagcttccagggggaaacgcctggtatctttatagtcctgtcgggtttcgccacctctgactt
gagcgtcgatttttgtgatgctcgtcaggggggcggagcctatggaaaaacgccagcaacgcggcctttttacggttc
ctggccttttgctggccttttgctcacatagtcatgccccgcgcccaccggaaggagctgactgggttgaaggctctc
aagggcatcggtcgagatcccggtgcctaatgagtgagctaacttacattaattgcgttgcgctcactgcccgctttcc
agtcgggaaacctgtcgtgccagctgcattaatgaatcggccaacgcgcggggagaggcggtttgcgtattgggcg
ccagggtggtttttcttttcaccagtgagacgggcaacagctgattgcccttcaccgcctggccctgagagagttgca
gcaagcggtccacgctggtttgccccagcaggcgaaaatcctgtttgatggtggttaacggcgggatataacatgag
ctgtcttcggtatcgtcgtatcccactaccgagatatccgcaccaacgcgcagcccggactcggtaatggcgcgcatt
gcgcccagcgccatctgatcgttggcaaccagcatcgcagtgggaacgatgccctcattcagcatttgcatggtttgtt
gaaaaccggacatggcactccagtcgccttcccgttccgctatcggctgaatttgattgcgagtgagatatttatgcca
gccagccagacgcagacgcgccgagacagaacttaatgggcccgctaacagcgcgatttgctggtgacccaatgc
gaccagatgctccacgcccagtcgcgtaccgtcttcatgggagaaaataatactgttgatgggtgtctggtcagagac
atcaagaaataacgccggaacattagtgcaggcagcttccacagcaatggcatcctggtcatccagcggatagttaat
gatcagcccactgacgcgttgcgcgagaagattgtgcaccgccgctttacaggcttcgacgccgcttcgttctaccat

cgacaccaccacgctggcacccagttgatcggcgcgagatttaatcgccgcgacaatttgcgacggcgcgtgcag
ggccagactggaggtggcaacgccaatcagcaacgactgtttgcccgccagttgttgtgccacgcggttgggaatgt
aattcagctccgccatcgccgcttccactttttcccgcgttttcgcagaaacgtggctggcctggttcaccacgcggga
aacggtctgataagagacaccggcatactctgcgacatcgtataacgttactggtttcacattcaccaccctgaattga
ctctcttccgggcgctatcatgccataccgcgaaaggttttgcgccattcgatggtgtccgggatctcgacgctctccc
ttatgcgactcctgcattaggaagcagcccagtagtaggttgaggccgttgagcaccgccgccgc

### INDUSTRAIL APPLICABILITY

The present disclosure discloses a recombinant vector of a thermolabile UNG fused protein and an expressing and purifying method. The method comprises cloning a Cod UNG genetic sequence to a pCold-SUMO vector to construct a recombinant vector pCold-SUMO-Cod UNG, transforming the recombinant vector to E. coli BL21 (DE3) competence, transforming and expressing molecular chaperone plasmids pG-Tf2 into the competence, inducing the expression at a low temperature to obtain a soluble SUMO-Cod UNG fused protein. The present disclosure resolves an insoluble problem of a Cod UNG expression product, it has industrial applicability.

## Claims

1. A recombinant vector containing a rCod UNG hydrolase, a sequence of the recombinant vector is SEQ ID NO. 5.

2. A method for expressing and purifying a thermolabile rCod UNG enzyme, **characterized in that** it comprises the following steps:
1) preparing a recombinant vector containing rCod UNG hydrolase; the vector has a His tag and a sumo tag; the recombinant vector is introduced into Escherichia coli BL21 (DE3) containing a pG-Tf2 molecular chaperone plasmid;
2) inducing expression; and
3) purifying: purifying a fused protein by the His tag to obtain rCod UNG enzyme with a high purity.

3. The method for expressing and purifying the thermolabile rCod UNG enzyme according to claim 2, **characterized in that** a nucleotide sequence for coding the rCod UNG hydrolase is SEQ ID NO. 3; and a sequence of the recombinant vector is SEQ ID NO. 5.

4. The method for expressing and purifying the thermolabile rCod UNG enzyme according to claim 2, **characterized in that** in step 2, a medium for inducing the expression is 1 weight/volume % of NaCl; 1 weight/volume % of tryptone; and 0.5 weight/volume % of yeast extract.

5. The method for expressing and purifying the thermolabile rCod UNG enzyme according to claim 2, **characterized in that** conditions for inducing the expression are that OD₆₀₀ is 0.5-0.7, 0.25 mM of IPTG is added, and the expression is induced for 16 hours at 15 °C and 230 rpm.

6. The method for expressing and purifying the thermolabile rCod UNG enzyme according to claim 2, **characterized in that** the purifying comprises lysing strains, extracting the fused protein, and purifying the fused protein.

7. The method for expressing and purifying the thermolabile rCod UNG enzyme according to claim 6, **characterized in that** lysing the strains comprises collecting the strains by a first centrifugation of a strain solution obtained by inducing the expression, resuspending by a precooled binding buffer, deconstructing by sonication in an ice-water bath, performing a second centrifugation after the sonication is complete, taking a supernatant after the centrifugation, performing a third centrifugation, taking a supernatant, adding polyethyleneimine into the supernatant, culturing for 15-20 minutes, and performing a fourth centrifugation;
conditions of the first centrifugation are centrifugating for 10 minutes at 2500 g and 4 °C;
conditions of the second centrifugation are centrifugating for 10 minutes at 11000 g and 4 °C;
conditions of the third centrifugation are centrifugating for 10 minutes at 1100 g and 4 °C; and
conditions of the fourth centrifugation are centrifugating for 15 minutes at 1100 g and 4 °C.

8. The method for expressing and purifying the thermolabile rCod UNG enzyme according to claim 7, **characterized in that**
a volume ratio of the strain solution obtained by inducing the expression to the binding buffer is 20:1;
a power for deconstructing by the sonication is 50%, a frequency is sonicating for 2 seconds and stopping for 3 seconds, and a duration is 20-25 minutes; and
a volume ratio of the polyethyleneimine to a filtration solution is 1:1000-2000, preferably 1:1000.

9. The method for expressing and purifying the thermolabile rCod UNG enzyme according to claim 2, **characterized in that** extraction steps of the fused protein comprise: adding a supernatant into an Ni-NTA protein extraction column until all of the supernatant flows through Ni-beeds; then adding a wash buffer having a same volume as the Ni-beeds to wash off impure proteins by repeating 1-2 times;
then adding an elution buffer for an elution, repeating the elution 4-6 times, and collecting all eluates.

10. The method for expressing and purifying the thermolabile rCod UNG enzyme according to claim 2, wherein the purifying the fused protein comprises dialyzing twice with a storage solution for the rCod UNG protein.
